# EUROPEAN PATENT SPECIFICATION

(11) **EP 0 302 887 B1**
(45) Date of publication and mention of the grant of the patent: **22.06.1994**
(21) Application number: 87903113.6
(22) Date of filing: 14.04.1987
(51) Int. Cl.: C12P 19/04, A61K 39/02, C08B 37/00, C07K 17/02

(54) **BACTERIAL ANTIGENS, ANTIBODIES, VACCINES, AND METHODS OF MANUFACTURE**
BAKTERIELLE ANTIGENE, ANTIKÖRPER, IMPFSTOFFE UND DEREN HERSTELLUNG
ANTIGENES, ANTICORPS, VACCINS BACTERIENS ET LEUR PROCEDE DE PRODUCTION

(30) Priority: 16.04.1986 US 852840
(43) Date of publication of application: 15.02.1989
(60) Divisional application: 93202308.8
(73) Proprietor: THE BRIGHAM AND WOMEN'S HOSPITAL, INC., Boston, MA 02115 (US)
(72) Inventor: KASPER, Dennis, L., Newton Center, MA 02159 (US); JENNINGS, Harold, J., Gloucester Ontario KIJ 6G6 (CA); LEVY, Nancy, J., Newton Center, MA 02159 (US); WESSELS, Michael, R., Brookline, MA 02146 (US)
(74) Representative: Bizley, Richard Edward
(86) International application number: US8700845
(87) International publication number: WO8706267

(56) References cited:
- US-A- 4 207 414
- US-A- 4 356 263
- US-A- 4 413 057
- Biochemistry, vol. 20, no.16, issued 4 August 1981, H.J. Jennings et al "Conformational aspects critical to the immunospecificity of the Type III streptococcal polysaccharide", pages 4511-4518
- Canadian Journal of Biochemistry, vol. 58, issued 1980, H.J. Jennings et al "Structural determination and serology of the native polysaccharide antigen of type-III group B Streptococcus", pages 112-120
- The Journal of Biological Chemistry, vol. 256, no.8, issued 25 April 1981, M.Kitamikado et al "Isolation and characterization of a Keratin sulfate-degrading Endo-B-galactosidase from Flavobacterium keratolyticus, pages 3906-3909
- The Journal of Experimental Medicine, vol. 163, no.2, issued 1 February 1986, R.A. Insel et al "Oligo-saccharide-protein conjugate vaccines induce and prime for oligoclonal IgG antibody responses to the Hemophilus influenzae b Capsular polysaccharide in human infants", pages 262-269

## Description

This invention relates to antigens having immunogenic determinants of Group B Streptococcus bacteria, to vaccines protecting against those bacteria, to methods of making such vaccines, and to methods of preparing oligosaccharides from bacterial polysaccharides. As used in this application, the term Group B Streptococcus (or GBS) bacteria is used as understood by those in the field, particularly with reference to Lancefield, J. Exp. Med. 108:329-341 (1938) and subsequent work further characterizing Group B serotypes, e.g. Russell-Jones, J. Exper. Med. 160:1476 (1984). The term specifically includes bacteria taxonomically designated Streptococcus agalactiae.

GBS are a recognized etiological agent for bacteremia and/or meningitis in infants, and for infections in adults. Baker, "Group B Streptococcal Infections" in Advances in Internal Medicine, 25:475-500 (1980). Accordingly, it is important to develop rapid and definitive assays for diagnosis of GBS infection, and methods of generating protection against GBS, particularly in infants and compromised individuals.

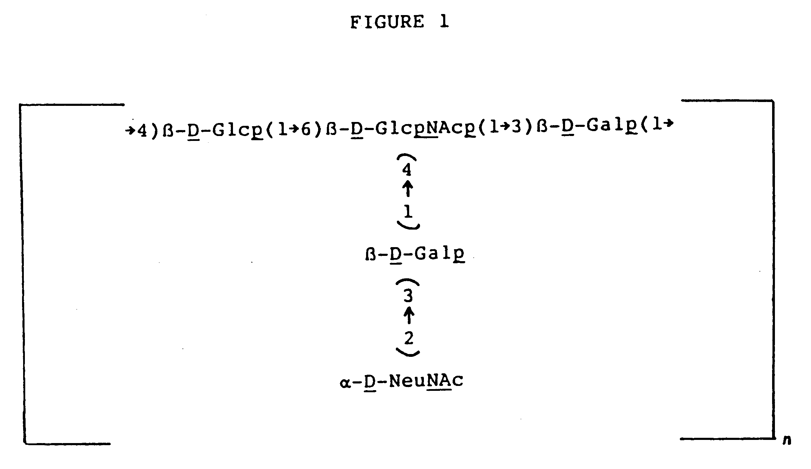

The GBS capsular polysaccharides are known to be important to GBS virulence and immunity. Baker, cited above. Moreover, the recognized GBS types and subtypes have chemically related but antigenically distinct capsular polysaccharides having a repeating structure composed of galactose, glucose, N-acetyl glucosamine, and N-acetyl-neuraminic (sialic) acid. Baker, cited above. Type III GBS capsular polysaccharide is composed of a backbone made up of repeating branched pentasaccharide units, as shown in Fig 1. Jennings et al., Canadian J. Biochem. 58:112-120 (1980).

One study of type III GBS polysaccharides suggests that the natural immunodeterminant site is located at the side chain-backbone junction. Jennings et al., Biochemistry 20:4511-4518 (1981). The presence of the side chain terminal N-acetyl-neuraminic acid residues reportedly was critical for immunodeterminant expression.

Insel and Andersen, J. Exp. Med. 163:262 (1986) disclose conjugating Haemoplilus influenzae capsular polysaccharide to a protein carrier in an effort to convert the capsule to a more thymus-dependent immunogen.

We have discovered that oligomers of the repeating pentasaccharide unit of the type III Group B. Streptococcus (III-GBS) polysaccharide capsule (and even the single unit alone) are antigenic. Antigens containing the unit, particularly antigens containing repeated units, can be used as a component of a III-GBS vaccine. Accordingly, one aspect of the invention features an antigen capable of raising an antibody selectively immunoreactive with type III Group B Streptococcus (III GBS) bacteria, and comprising a purified oligosaccharide hapten and a carrier associated with said oligosaccharide hapten having the following formula: 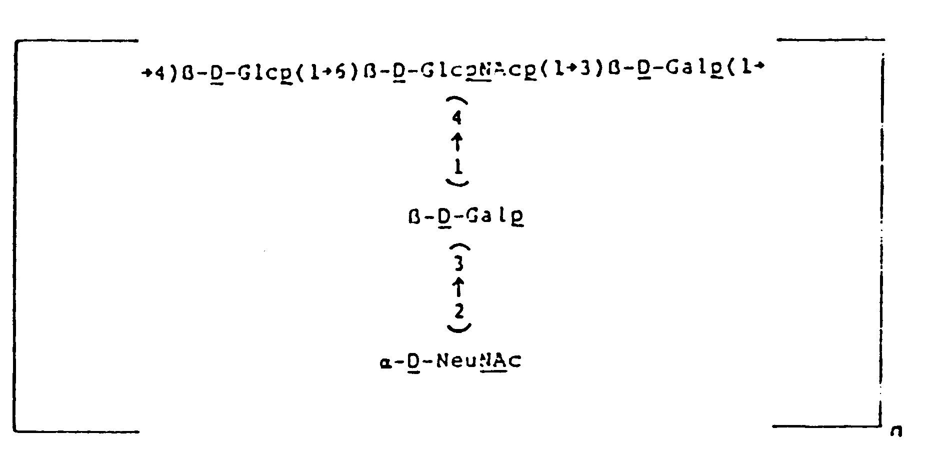
in which n=1-50, GlcNAcp represents N-acetyl glucosamine (in the pyranose form), Galp represents galactose (in the pyranose form), Glcp represents glucose (in the pyranose form), and ^{**α**}-D-NeuNAc represents N-acetyl neuraminic (sialic) acid.

"Purified" means substantially separated from the various protein, lipid, and carbohydrate components that naturally occur with the oligosaccharide. In particular, purified oligosaccharide is substantially free from intact III GBS polysaccharide capsule, or fragments of it having a molecular weight above 100,000. Whatever traces of foreign components are in the purified oligosaccharide do not interfere with the use of the purified material in a vaccine or as an antigen. The term "purified" is not intended to exclude synthetic oligosaccharide preparations retaining artifacts of their synthesis; nor is the term meant to exclude preparations that include some impurities, so long as the preparation exhibits reproducible oligosaccharide characterization data, for example molecular weight, sugar residue content, sugar linkages, chromatographic response, and immunogenic behavior.

In a second aspect, the invention features a vaccine capable of eliciting protection against III GBS, comprising a pharmaceutically acceptable vehicle and the above described antigen, optionally conjugated to a carrier.

A third aspect of the invention features a method of making the above-described antigen by: 1) culturing III-GBS bacteria in a suitable medium; 2) recovering polysaccharide in the medium or from the bacteria cells; 3) digesting the polysaccharide with an endo-β-galactosidase specific for cleaving the linkage S₁(1^{→}3)-β-gal(1^{→}4)S₂, where S₁ and S₂ are independently selected from glucose, glucosamine, or N-acetyl glucosamine; and 5) recovering the oligosaccharide antigen from the medium or from the bacteria. Optionally, the oligosaccharide is conjugated to a carrier as described below.

In preferred embodiments of any of the first three aspects, the oligosaccharide antigen is produced by enzymatic hydrolysis of III GBS capsular polysaccharide using an endo-β-galactosidase specific for the above-described linkage. One such endo-β-galactosidase is found in Flavobacterium keratolyticus. Also preferably, the oligosaccharide is covalently bound (e.g., via a secondary amine link) to a carrier such as a protein, particularly a bacterial toxoid or a bacterial surface protein, comprising a bacterial immunodeterminant site. For example, the oligosaccharide may be covalently bound to a substantially purified trypsin-resistant C surface protein of type Ic Group B Streptococcus which has a molecular weight of about 14,000 and is non-cross-immunoreactive with group B Streptococcus bacterial polysaccharides yet cross-immunogenic with type Ia/c GBS.

In a fourth aspect, the invention more generally features enzymatic cleavage of a bacterial polysaccharide having the above-described linkage, in a method of making a purified oligosaccharide; specifically a bacteria having a surface polysaccharide (e.g. a polysaccharide capsule or membrane) is cultured, and the polysaccharide is extracted, after which it is digested with the above-described enzymes and the oligosaccharide is recovered. Preferably the bacteria is gram positive, e.g., type III GBS; alternatively the bacteria can be a species having a surface capsule polysaccharide, such as type 14 Streptococcus pneumoniae, e.g., ATCC No. 6314..

Also, the invention features a method of assaying a sample for anti GBS antibody by adding to the sample the oligosaccharide antigen described above, and then detecting the formation of an immunocomplex; alternatively a sample is assayed for the presence of a GBS immunodeterminant by raising an antibody to the oligosaccharide antigen, adding the antibody to the sample, and detecting the formation of an immunocomplex.

In yet another aspect, the invention features a gamma globulin fraction capable of passive protection against GBS, the fraction being produced by immunizing a mammal with the above-described vaccine. The fraction is then adminstered to an individual to provide protection against GBS infection or to treat on-going infection.

The enzymatic selective hydrolysis of the backbone glycosidic bonds is superior to simple acid hydrolysis, because the latter technique results in the loss of the side chain sialic acid residues, and cleaves bonds non-selectively, yielding a heterogeneous mixture of reduced immunogenicity. In contrast, enzymatic digestion preserves the sialic acid residues and produces backbone cleavage only at the gal-β 1-4-glc bonds, which occur once per backbone repeating unit.

Other features and advantages of the invention will be apparent from the following description of the preferred embodiments and from the claims.

We now describe preferred embodiments of the invention, first briefly describing the drawing.

### I. Drawing.

Fig. 1 is a diagram of a repeating pentasaccharide of the III GBS capsular polysaccharide.

### II. Type III GBS Antigen And Vaccine

### A. Oligosaccharide

The specific pentasaccharide described above is obtained by culturing type III GBS and extracting the polysaccharide capsule from the supernatant broth or from the bacterial cells by the general method of Jennings et al., Canadian J. Biochem. 58:112-120 (1980). The polysaccharide extract is digested by an endo-β-galactosidase that specifically cleaves the structure S₁(1^{→}3)βgal(1^{→}4)βS₂, where S₁, and S₂ are independently selected from glucose, glucosamine, or N-acetyl glucosamine. The above structure occurs once per repeating unit of the III GBS polysaccharide capsule, and the resulting cleavage products are oligosaccharides having one or more of the above-described pentasaccharide repeating units.

The enzyme is obtained from bacteria known to produce an enzyme catalyzing the requisite specific cleavage. One such preferred bacteria is Flavobacterium keratolyticus. Another bacterium thought to produce an enzyme with the requisite specific activity is Bacteroides fragilis. Kitamikado et al., cited below, report that the requisite endo-β-galactosidase activity is also present in Escherichia freundii, Pseudomonas sp., and Cocobacillus sp. when induced on keratan sulfate. See Nakagawa et al. J. Biol. Chem. 250:912-917; and Hirano et al. Connect. Tissue Res. 2:1-10. A suitable F. keratolyticus can be obtained from the Institute of Fermentation (IFO) Osaka, Japan, under the designation IFO #14087. A suitable general procedure for preparing endo-β-galactosidase is given by Kitamikado et al., J. Biol. Chem. 2:3906-3909 (1981).

The extracted III GBS polysaccharide capsule prepared as described above is added to a buffered enzyme preparation, and incubated (e.g., 37°C, 48 hrs.). After digestion, the oligosaccharides are separated by molecular weight by gel filtration chromatography, e.g., Sephadex G75 (Pharmacia), and purified by anion exchange chromatography, e.g., anion exchange high performance liquid chromatography (HPLC), Mono Q (Pharmacia), or QAE Sephadex (Pharmacia).

A purified oligosaccharide comprising between about 1 and 50 (and most preferably between about 2 and 30 pentasaccharide units) is selected from chromatographic group and then conjugated to a protein for use in a vaccine. The protein may be an inactive carrier, or it can be selected to provide some protection in its own right, particularly protection that complements the protection induced by the oligosaccharide, for example, by inducing protection against other types of GBS or other bacteria. The non-toxic diphtheria toxin analog, CRM 197, may be used as described by Insel et al. (1986) J. Exp. Med. 163:262 and Andersen et al. J. Clin. Invest. 76:52 (1985). Other toxoids that can be used include tetanus or standard diphtheria toxoids available from the Massachusetts State Laboratory, South Street, Jamaica Plain, MA.

Protein-oligosaccharide conjugation is achieved by a coupling reaction, such as the general method of Swartz and Gray, Arch. Bioch. Biophys. (1977) 181:542, which couples a reducing sugar directly to a protein. Oligosaccharide fragments produced by deamination can be directly linked via cyanoborhydride to the carrier protein. In the case of III GBS, a secondary amine is formed by bonding between a nitrogen on the protein and an aldehyde group on 2,5-anhydromannose formed by deamination of the oligosaccharide. This aldehyde is available for direct coupling using cyanoborhydride.

The resulting oligosaccharide-protein conjugate is suspended in pyrogen-free saline or any other physiologically acceptable non-toxic vehicle or medium, which may contain any conventional stabilizers or other additives as desired. The concentration of antigen is not critical and may be varied over a wide range, but for many purposes a range of 10-1000 ^{»}g/ml is convenient and suitable. In this form it is stable during prolonged storage at 4°C. and is sterile, non-toxic, and non-pyrogenic when subjected to animal tests as prescribed by Food and Drug Administration Regulations (Title 21, Sec. 610.11-610.13). Suitable volumes (e.g. about 0.5 ml) of the above-described saline suspension are administered (e.g. by subcutaneous injection) to induce III GBS protection.

The invention also features a method of passive immunization, used particularly for infants or compromised adults, by injecting the oligosaccharide-protein conjugate vaccine into a human to raise antibodies thereto in high titer, separating the antiserum from the blood of the human, and fractionating the antiserum to produce a gamma globulin fraction containing the antibodies, which can be used for passive immunization. This method of establishing donors for passive immunization is useful because, although occasional non-immunized human adults have very high levels of type-specific GBS antibody in their sera, it would be necessary to screen very large populations to select those individuals whose plasma could be pooled to make sufficiently high titered globulin fractions to be useful for passive immunizations. Several lots of pooled human gamma globulin from non-immunized adults have been assayed for anti-Types Ia and Ic, anti-Type II, and anti-Type III polysaccharide antibody and have been found to contain only 5-20 ^{»}g/ml of serum. Immunizations by intravenous injection of such low titer globulins would require prohibitively large doses with attendant risk of adverse reactions.

For passive immunization, pools of human sera from selected individuals vaccinated with the above-described conjugated polysaccharide vaccine can be concentrated and fractionated by conventional procedures to provide a globulin fraction containing most of the type-specific antibody and of sufficiently high activity so that the hyperimmune globulin is effective in small doses of 0.3-1 ml, preferably about 0.5 ml, when administered in a suitable physiologically acceptable carrier such as normal saline either intravenously or intramuscularly. The concentration of the globulin in the carrier may be from 5 to 20% by weight. The hyperimmune globulin can be administered either to pregnant women prior to delivery, to neonates, or to immunologically compromised individuals, to provide passive immunization or therapy.

### III. Oligosaccharide Antibodies

Oligosaccharides generally can be recovered from endo-β-galactosidase digestion of baterial polysaccharides. The above description concerns III GBS oligosaccharides and their use in vaccines, however, the oligosaccharides also can be used to elicit antibody in experimental mammals, e.g., by challenging the mammal, e.g. by injecting the oligosaccharide protein conjugates, and recovering the resulting antibody. Also, monoclonal antibodies can be generated by standard techniques. The resulting antibodies are useful, e.g. in immunoassays for GBS, or III GBS in particular. For example, the antibody to the oligosaccharide can be used for immunoassays such as competitive or sandwich immunoassays known in the field.

The following examples illustrate the oligosaccharide antigen, the method of isolating the oligosaccharide, the vaccine, and the method of passive immunization.

### Example 1: Preparation of endo-β-galactosidase

F. keratolyticus is cultured overnight and used to inoculate a modified trypticase peptone broth (3% trypticase peptone from BBL Microbiology Systems, Cockeysville, MD); 0.1% yeast extract (Difco Laboratories, Detroit MI); 0.2% NaCl, pH7.0. The broth is incubated for 18 hours (Biolafitte fermentor, Lafitte, France), while controlling aeration (15 l/min), stirring (150 rpm), temperature (25°C), and pH (7.0). Bacteria are removed by centrifugation and the supernatant concentrated (Pellicon Cassette System, Millipore Corp., Bedford MA). Solid (NH₄)₂SO₄ is added to 75% saturation and the solution is allowed to stand overnight at 4°C.

Precipitate is removed by centrifugation and dissolved in 10mm sodium acetate, 0.2M NaCl, pH6.0. The solution is loaded onto a 5 x 90 cm Sephadex G-100 column (Pharmacia Fine Chemicals, Piscataway NJ) and eluted with the same buffer at 40ml/hr, 4°C. Active fractions are identified by assaying for endo-β-galactosidase activity and then pooled. Specifically, the fractions are incubated with bovine cornea keratan sulfate (Sigma Chemical Co., St. Louis MO) overnight at 37°C. Production of reducing sugar is measured by the general method of Park and Johnson, J. Biol. Chem. 181:149-151 (1949).

Pooled active fractions are dialyzed, lyophilized, and dissolved in buffer (50 mM sodium acetate, 2mM CaCl₂, pH6.0). The buffered enzyme preparation is loaded onto a 2.5 x 14 cm column containing DEAE Sephacel (Pharmacia) in the top half and BioRex 70 (BioRad Laboratories, Rockville Center NY) in the bottom half. The enzyme elutes with 250ml of the above buffer. Residual bound protein is eluted with 1.0M NaCl, and active pooled fractions are dialyzed against 10mM sodium acetate, 2mM CaCl₂, pH7.0, and lyophilized.

### Example 2: Polysaccharide Digestion

GBS type III polysaccharide is extracted by the general technique of Jennings (1980) cited above. The lyophilized endo-β-galactosidase preparation from one DEAE Sephacel/BioRex 70 column run is dissolved in 10ml 10mM sodium acetate, 2mM calcium chloride, pH7.0 and dialyzed against 2 liters of the same buffer overnight at 4°C with one bath exchange. Twenty mg of purified type III GBS capsular polysaccharide is added to the enzyme preparation. The mixture is filter sterilized, and incubated at 37°C for 48 h, with stirring.

### Example 3: Oligosaccharide Purification And Characterization

After digestion, oligosaccharides are separated into large versus small molecular weight pools by dialysis against water through a 12,000-14,000 dalton pore size membrane (Spectrum Medical Industries, Los Angeles, CA). Oligosaccharides corresponding to one (pentasaccharide) and two (decasaccharide) repeating units are purified by anion exchange chromatography. Small amounts of oligosaccharides (1 mg or less) can be purified by anion exchange high performance liquid chromatography (HPLC). Five hundred ^{»}g of digestion mixture (small molecular weight pool) are loaded on a 5 x 50 mm Mono Q column (Pharmacia) and one and two repeating unit oligosaccharides are eluted isocratically with 20 mM Tris HCl buffer, pH 7.2. Larger oligosaccharides can be eluted with higher salt concentrations. For purification of large amounts of oligosaccharides, a 1.2 x 10 cm column filled with QAE Sephadex A50 (Pharmacia) is used. Samples of 2-8 mg are loaded onto this column in a starting buffer of 20 mM N-methyl diethanolamine acetate, pH 9.6. The column is washed with 30 ml starting buffer at 1 ml/min, then the single repeating unit oligosaccharide is eluted with 50 ml of starting buffer containing 15 mM Na acetate. Two ml fractions are collected, neutralized with 1M acetic acid, and analyzed by thin layer chromatography (TLC). Fractions containing the single repeating unit oligosaccharide are pooled, lyophilized, dissolved in 2 ml water, and desalted on a 1.6 x 25 cm column containing Sephadex G15 (Pharmacia).

The major digestion product corresponds to a band migrating between tetrasaccharide and decasaccharide standards, and has a molecular size compatible with that predicted for the pentasaccharide unit of Fig. 1. Diphenylamine and resorcinol staining of this band is consistent with a sialylated sugar. Methylation analysis confirms that structure is a complete pentasaccharide repeating unit.

One and two repeating-unit oligosaccharides are purified by anion exchange chromatography. Anion exchange HPLC is a quick and convenient method for purifying small amounts of oligosaccharides (1-2 mg or less). The single repeating unit oligosaccharide elutes in the void volume, while the two repeating unit oligosaccharide elutes later in the isocratic phase. Larger oligosaccharides or native polysaccharide, are retained in the isocratic phase, but are readily eluted from this column with 0.5 M sodium chloride. This method can be used to purify tritiated one and two repeating unit oligosaccharides for use in radioantibody binding assay (RABA) studies. See Schifferle (1985) J. Immunology 135:4164. For purification of larger amounts of single repeating unit oligosaccharide, an open anion exchange column can be used to provide a larger binding capacity. Using this QAE Sephadex A50 column, the single repeating unit, as well as larger oligosaccharides are retained in the starting buffer. The single repeating unit is eluted, free of larger oligosaccharides, with the starting buffer containing 15mM sodium acetate. Larger oligosaccharides remain bound, and can be eluted with increased salt concentration. From digestion of 20 mg of type III native polysaccharide, 6 mg of purified single repeating unit oligosaccharide are obtained. The oligosaccharide corresponding to a minor band migrating just distal to the single repeating unit on TLC is also retained somewhat more avidly by the anion exchange column. Although complete separation is not achieved with preparative runs, the single repeating unit oligosaccharide purified in this manner is estimated to be 95% pure by TLC and methylation anaylsis. Larger oligosaccharides are also fractionated by chromatography, e.g., a Sephadex G75 column.

### Example 4: Binding Assay For Oligosaccharide

The chromatogram binding assay is used to examine directly the ability of the oligosaccharide bands visualized by TLC to bind antibody directed against the native polysaccharide. Antibody binding to the single repeating unit band is clearly evident. Although the assay is not quantitative, it is clear from the relative intensities of the bands on the autoradiograph that the native polysaccharide binds antibody more efficiently. This assay is highly specific for native type III polysaccharide--cross reactions are not seen with the desialylated core polysaccharide, with the group B polysaccharide, nor with capsular polysaccharides from other GBS serotypes.

The oligosaccharide antigen described above is then conjugated to a protein as described above, and suspended in pyrogen-free saline solution, as described above, to form a vaccine.

The single repeating pentasaccharide (Fig. 1) is weakly antigenic using radioantibody binding assay inhibition TLC binding assay. and direct radioantibody binding assay. Increasing oligosaccharide size to two repeating units results in an eight-fold increase in antigen binding. Most preferably, the immunogen includes 2-50 units.

## Claims

1. An immunogenic substance, raising an antibody selectively immunoreactive with type III Group B Streptoccocus (III GBS) bacteria, said immunogenic substance comprising a purified oligosaccharide hapten, and a carrier associated with said oligosaccharide hapten, said oligosaccharide hapten having the formula: 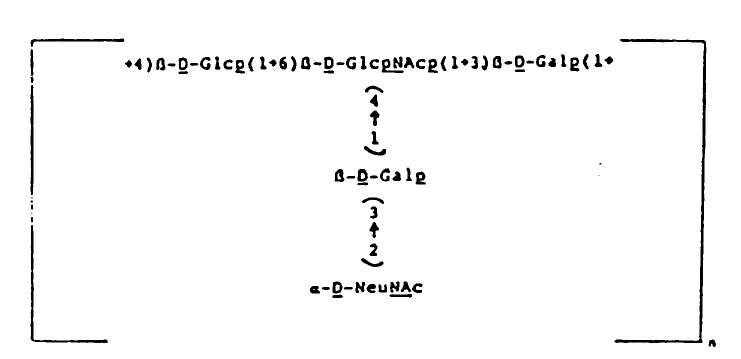 in which n=1-50 GlcNAcp represents N-acetyl glucosamine (in the pyranose form), Galp represents galactose (in the pyranose form), Glcp represents glucose (in the pyranose form), and α-D-NeuNAc represents N-acetyl neuraminic (sialic) acid.

2. The immunogenic substance of claim 1 wherein said oligosaccharide hapten is obtained by enzymatic hydrolysis of III GBS polysaccharide capsule having a backbone and side chains, using endo-β-galactosidase specific for cleaving one linkage on the backbone of said III GBS polysaccharide without cleaving side chain linkages of said III GBS polysaccharide and without cleaving other backbone linkages of said III GBS polysaccharide.

3. The immunogenic substance of claim 2 wherein said endo-β-galactosidase is obtained from Flavobacterium keratotylicus.

4. The immunogenic substance of claim 1 wherein said oligosaccharide is covalently bound to a protein.

5. The immunogenic substance of claim 4 wherein said protein is covently bound to said oligosaccharide by a secondary amine function.

6. The immunogenic substance of claim 4 wherein said protein comprises a bacterial surface protein.

7. The immunogenic substance of claim 6 wherein said bacterial surface protein is a substantially purified trypsin-resistant C surface protein of type Ic group B Streptococcus having a molecular weight of about 14,000 which is non-cross-immunoreactive with group B Streptococcus bacterial polysaccharides, yet cross-immunogenic with type Ia/c GBS.

8. The immunogenic substance of claim 4 wherein said protein comprises a bacterial toxoid.

9. The immunogenic substance of claim 1 substantially free from intact III GBS polysaccharide capsule, or fragments of it having molecular weight above 100,000.

10. A method of making the immunogenic substance of claim 1 comprising:
a) culturing a type III group B Streptococcus bacterium;
b) recovering polysaccharide capsule in said medium or bacterial cells, said polysaccharide capsule having a backbone and side chains;
c) digesting said polysaccharide with an endo-β-galactosidase specific for cleaving one linkage on the backbone of said III GBS polysaccharide without cleaving side chain linkages of said III GBS polysaccharide and without cleaving other backbone linkages of said III GBS polysaccharide;
d) recovering said oligosaccharide hapten; and
e) associating said oligosaccharide hapten with a carrier.

11. The method of claim 10 wherein said endo-β-galactosidase is selected to cleave the D-Galp(1-4)β-D-Glcp backbone III GBS linkage, without cleaving the III GBS side chain linkages.

12. The method of claim 10 wherein said endo-β-galactosidase is obtained from Flavobacterium keratolyticus.

13. The method of claim 10 wherein said method comprises covalently binding said oligosaccharide to a protein.

14. The method of claim 13 wherein said protein is covalently bound to said oligosaccharide by a secondary amine function.

15. The method of claim 13 wherein said protein comprises a bacterial surface protein.

16. The method of claim 13 wherein said protein comprises a bacterial toxoid.

17. A vaccine that elicits immunological protection against type III group B Streptoccocus bacteria, said vaccine comprising a pharmaceutically acceptable vehicle and the immunogenic substance of any one of claims 1 to 9.

18. A method of making a purified oligosaccharide comprising,
culturing a bacterium comprising a surface polysaccharide having at least one linkage S₁(1-3)βgalβ(1-4)S₂ where S₁ and S₂ are independently selected from the group consisting of glucose, glucosamine, and N-acetyl glucosamine,
extracting said surface polysaccharide, digesting said polysaccharide with an endo-β-galactosidase specific for said linkage without cleaving III GBS side chain linkages; and
recovering said purified oligosaccharide.

19. The method of claim 18 wherein said bacterium is a gram positive bacterium and said surface polysaccharide is a capsular polysaccharide.

20. The method of claim 19 wherein said bacterium is a type III Group B Streptococcus bacterium.

21. The method of claim 18 wherein said bacterium is type 14 Streptococcus pneumoniae.

22. Use of the immunogenic substance of any one of claims 1 to 9 in a method of assaying a sample of anti GBS antibody comprising adding to said sample said immunogenic substance and detecting formation of an immunocomplex.

23. Use of the immunogenic substance of any one of claims 1 to 9 in a method of assaying a sample for the presence of a GBS immunodeterminant comprising
raising an antibody to the said immunogenic substance,
adding said antibody to said sample, and
detecting the formation of an immunocomplex.

## Patentansprüche

1. Immunogene (= Immunreaktion auslösende) Substanz, die zum Heranziehen eines Antikörpers, der mit dem Typ III-Streptococcus-Bakterium der Gruppe B (III GBS) selektiv immunoreaktiv ist, führt, wobei die immunogene Substanz ein gereinigtes Oligosaccharid-Hapten und einen Träger, der mit dem Oligosaccharid-Hapten assoziiert ist, umfäßt, wobei das Oligosaccharid-Hapten folgende Formel besitzt: 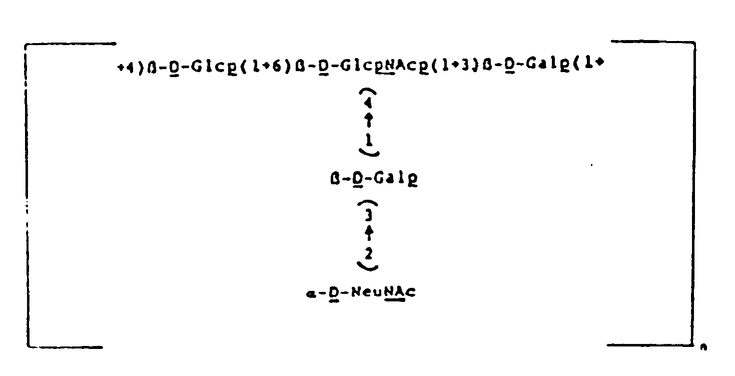 in welcher n = 1 - 50 ist, GlcNAcp für N-Acetylglucosamin (in der Pyranoseform) steht, Galp Galactose (in der Pyranoseform) bedeutet, Glcp für Glucose (in der Pyranoseform) steht, und α-D-NeuNAc für N-Acetylneuramin(Sialin)-säure steht.

2. Immunogene Substanz nach Anspruch 1, bei der das Oligosaccharid-Hapten durch enzymatische Hydrolyse von einer ein Grundgerüst und Seitenketten aufweisenden III-GBS-Polysaccharidkapsel erhalten wird, und zwar durch Anwendung einer endo-β-Galactosidase, die für die Spaltung einer Bindung am Grundgerüst des III-GBS-Polysaccharids ohne Spaltung der Seitenkettenbindungen des III-GBS-Polysaccharids und ohne Spaltung anderer Grundgerüstbindungen des III-GBS-Polysaccharids spezifisch ist.

3. Immunogene Substanz nach Anspruch 2, bei der die endo-β-Galactosidase aus Flavobacterium keratolyticus erhalten wird.

4. Immunogene Substanz nach Anspruch 1, in der das Oligosaccharid kovalent an ein Protein gebunden ist.

5. Immunogene Substanz nach Anspruch 4, in der das Protein kovalent an das Oligosaccharid durch eine sekundäre Aminfunktion gebunden ist.

6. Immunogene Substanz nach Anspruch 4, bei der das Protein ein bakterielles Oberflächenprotein enthält.

7. Immunogene Substanz nach Anspruch 6, bei der das bakterielle Oberflächenprotein ein im wesentlichen gereinigtes Trypsin-resistentes C-Oberflächenprotein vom Typ Ic der Streptococcus-Gruppe B mit einem Molekulargewicht von etwa 14 000, welches nicht kreuzimmunoreaktiv mit bakteriellen Polysacchariden der Streptococcus-Gruppe B, aber noch kreuz-antigen mit dem Typ Ia/c GBS ist.

8. Immunogene Substanz nach Anspruch 4, bei der das Protein ein bakterielles Toxoid umfaßt.

9. Immunogene Substanz nach Anspruch 1, die im wesentlichen frei von intakten IIIGBS-Polysaccharidkapseln oder frei von Fragmenten davon mit einem Molekulargewicht über 100.000 ist.

10. Verfahren zur Herstellung der immunogenen Substanz nach Anspruch 1, umfassend:
(a) Kultivierung eines Typ III-Streptococcus-Bakteriums der Gruppe B;
(b) Wiedergewinnung der Polysaccharidkapsel in dem Medium oder den Bakterienzellen, welche Polysaccharidkapsel ein Grundgerüst und Nebenketten aufweist;
(c) Verdauung des Polysaccharids mit einer endo-β-Galactosidase, die für die Spaltung einer Bindung auf dem Grundgerüst des III-GBS-Polysaccharids ohne Spaltung von Nebenkettenbindungen des III-GBS-Polysaccharids und ohne Spaltung von anderen Grundgerüstbindungen des III-GBS-Polysaccharids spezifisch ist;
(d) Wiedergewinnung des Oligosaccharid-Haptens; und
(e) Assoziieren des Oligosaccharid-Haptens mit einem Träger.

11. Verfahren nach Anspruch 10, wobei die endo-β-Galactosidase augewählt wird, um die D-Galp(1-4)β-D-Glcp-III-GBS-Grundgerüstbindung zu spalten, ohne Spaltung der III-GBS-Nebenkettenbindungen.

12. Verfahren nach Anspruch 10, bei dem die endo-β-Galactosidase aus Flavobacterium keratolyticus erhalten wird.

13. Verfahren nach Anspruch 10, welches Verfahren das kovalente Binden des Oligosaccharids an einem Protein umfaßt.

14. Verfahren nach Anspruch 13, bei dem das Protein mittels einer sekundären Aminfunktion kovalent am Oligosaccharid gebunden wird.

15. Verfahren nach Anspruch 13, bei dem das Protein ein bakterielles Oberflächenprotein umfaßt.

16. Verfahren nach Anspruch 13, bei dem das Protein ein bakterielles Toxoid umfaßt.

17. Impfstoff, der ein immunologischen Schutz gegen Typ III-Streptococcus-Bakterien der Gruppe B hervorruft, wobei der Impfstoff ein pharmazeutisch annehmbares Vehikel und die immunogene Substanz gemaß mindestens einem der Ansprüche 1 bis 9 umfaßt.

18. Verfahren zur Herstellung eines gereinigten Oligosaccharids, umfassend:
Kultivierung eines Bakteriums, das ein Oberflächenpolysaccharid mit mindestens einer S₁(1-3)βgalβ(1-4)S₂-Bindung enthält, wobei S₁ und S₂ unabhängig aus der aus Glucose, Glucosamin und N-Acetylglucosamin bestehenden Gruppe ausgewählt werden;
Extrahieren des Oberflächenpolysaccharids, Verdauen des Polysaccharids mit einer endo-β-Galactosidase, die für die Bindung spezifisch ist, ohne das die III-GBS-Nebenkettenbindungen gespalten werden; und
Wiedergewinnung des gereinigten Oligosaccharids.

19. Verfahren nach Anspruch 18, bei dem das Bakterium ein grampositives Bakterium ist, und das Oberflächenpolysaccharid ein kapsuläres Polysaccharid ist.

20. Verfahren nach Anspruch 19, bei dem das Bakterim ein Typ III-Streptococcus-Bakterium der Gruppe B ist.

21. Verfahren nach Anspruch 18, bei dem das Bakterium Streptococcus pneumoniae vom Typ 14 ist.

22. Verwendung der immunogenen Substanz nach mindestens einem der Ansprüche 1 bis 9 bei einem Verfahren zur Untersuchung einer Anti-GBS-Antikörperprobe, umfassend das Hinzusetzen der immunogenen Substanz zu der Probe und Detektion der Bildung eines Immunkomplexes.

23. Verwendung der immunogenen Substanz nach mindestens einem der Ansprüche 1 bis 9 bei einem Verfahren zur Untersuchung einer Probe auf Anwesenheit einer GBS-Immundeterminante, umfassend:
Heranziehen eines Antikörpers gegen die immunogene Substanz,
Zugeben des Antikörpers zu der Probe, und
Detektieren der Bildung eines Immunkomplexes.

## Revendications

1. Substance immunogène, provoquant la formation d'un anticorps sélectivement immunoréactif avec des bactéries Streptococcus du groupe B type III (III GBS), ladite substance immunogène comprenant un haptène oligosaccharidique purifié, et une molécule porteuse associée à l'haptène oligosaccharidique, ledit haptène oligosaccharidique répondant à la formule 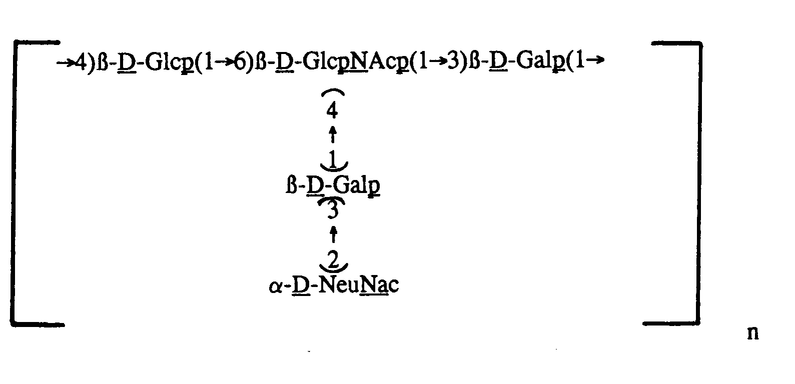 dans laquelle n=1-50 GLcNAcp représente une N-acétylglucosamine (sous la forme pyranose), Galp représente un galactose (sous la forme pyranose), Glcp représente un glucose (sous la forme pyranose), et α-D-NeuNac représente un acide N-acétylneuraminique (sialique).

2. Substance immunogène selon la revendication 1, dans laquelle ledit haptène oligosaccharidique est obtenu par hydrolyse enzymatique d'une capsule polysaccharidique de III GBS ayant un squelette et des chaînes latérales, en utilisant une endo-β-galactosidase spécifique pour rompre une liaison sur le squelette dudit polysaccharide de III GBS sans rupture des liaisons des chaînes latérales dudit polysaccharide de III GBS et sans rupture des autres liaisons du squelette dudit polysaccharide de III GBS.

3. Substance immunogène selon la revendication 2, dans laquelle ladite endo-β-galactosidase est obtenue à partir de Flavobacterium keratolyticus.

4. Substance immunogène selon la revendication 1, dans laquelle ledit oligosaccharide est lié de manière covalente à une protéine.

5. Substance immunogène selon la revendication 4, dans laquelle ladite protéine est liée de manière covalente au dit oligosaccharide par une fonction amine secondaire.

6. Substance immunogène selon la revendication 4, dans laquelle ladite protéine comprend une protéine de surface bactérienne.

7. Substance immunogène selon la revendication 6, dans laquelle ladite protéine de surface bactérienne est une protéine de surface résistante vis-à-vis de la trypsine de type C sensiblement purifiée de Streptococcus du groupe B type Ic possédant une masse moléculaire d'environ 14000,qui n'est pas immunoréactive de façon croisée avec les polysaccharides bactériens de Streptococcus du groupe B, tout en étant immunogène de façon croisée avec GBS type Ia/c.

8. Substance immunogène selon la revendication 4, dans laquelle ladite protéine comprend une anatoxine bactérienne.

9. Substance immunogène selon la revendication 1, sensiblement exempte de capsule polysaccharidique intacte de III GBS, ou de ses fragments possédant une masse moléculaire au-dessus de 100000.

10. Procédé de fabrication de la Substance immunogène selon la revendication 1, comportant les étapes de:
a) mise en culture d'une bactérie Streptococcus du groupe B type III
b) récupération de capsule polysaccharidique dans le milieu de culture ou dans les cellules bactériennes, ladite capsule polysaccharidique possédant un squelette et des chaînes latérales;
c) digestion dudit polysaccharide avec une endo-β-galactosidase spécifique pour rompre une liaison sur le squelette dudit polysaccharide de III GBS sans rupture des liaisons des chaînes latérales dudit polysaccharide de III GBS et sans rupture des autres liaisons du squelette dudit polysaccharide de III GBS;
d) récupération de l'haptène oligosaccharidique; et
e) association dudit haptène oligosaccharidique avec une molécule porteuse.

11. procédé selon la revendication 10, dans lequel ladite endo-β-galactosidase est choisie pour rompre la liaison D-Galp(1-4)β-D-Glcp du squelette de III GBS, sans rupture des liaisons des chaînes latérales de III GBS.

12. Procédé selon la revendication 10, dans lequel ladite endo-β-galactosidase est obtenue à partir de Flavobacterium keratolyticus.

13. Procédé selon la revendication 10, dans lequel ledit procédé comprend la liaison de manière covalente de l'oligosaccharide à une protéine.

14. Procédé selon la revendication 13, dans lequel ladite protéine est liée de manière covalente au dit oligosaccharide par une fonction amine secondaire.

15. Procédé selon la revendication 13, dans lequel ladite protéine comprend une protéine de surface bactérienne.

16. Procédé selon la revendication 13, dans lequel ladite protéine comprend une anatoxine bactérienne.

17. Vaccin provoquant une protection immunologique vis-à-vis des bactéries Streptococcus du groupe B type III, ledit vaccin comprenant un véhicule pharmaceutiquement acceptable et la substance immunogène selon l'une quelconque des revendications 1 à 9.

18. Procédè de fabrication d'un oligosaccharide purifié comportant les étapes de:
- mise en culture d'une bactérie comprenant un polysaccharide de surface ayant au moins une liaison S₁(1-3)βgalβ(1-4)S₂ où S₁ et S₂ sont choisis indépendamment dans le groupe composé de glucose, de glucosamine, et de N-acétylglucosamine;
- extraction dudit polysaccharide de surface, digestion dudit polysaccharide avec une endo-β-galactosidase spécifique de ladite liaison sans rupture des liaisons des chaînes latérales de III GBS; et
- récupération dudit oligosaccharide purifié.

19. Procédé selon la revendication 18, dans lequel ladite bactérie est une bactérie gram positif et ledit polysaccharide de surface est un polysaccharide capsulaire.

20. Procédé selon la revendication 19, dans lequel ladite bactérie est une bactérie Streptococcus du groupe B type III.

21. Procédé selon la revendication 18, dans lequel ladite bactérie est un Streptococcus pneumoniae du type 14.

22. Utilisation de la substance immunogène selon l'une quelconque des revendications 1 à 9 dans un procédé de test sur un échantillon d'anticorps anti GBS comprenant l'addition de ladite substance immunogène au dit échantillon et la détection de la formation d'un complexe immunologique.

23. Utilisation de la substance immunogène selon l'une quelconque des revendications 1 à 9 dans un procédé de test sur un échantillon pour détecter la présence d'un déterminant immunologique de GBS comportant les étapes:
- d'induction de la formation d'un anticorps dirigé contre ladite substance immunogène
- d'addition dudit anticorps au dit échantillon, et
- de détection de la formation d'un complexe immunologique.
